Europäisches Patentamt

European Patent Office

Office européen des brevets

(19)

(11) Publication number: **0 305 619 B1**

## EUROPEAN PATENT SPECIFICATION

(12)

(45) Date of publication of patent specification: **03.03.93**

(51) Int. Cl.⁵: **C12P 21/00**

(21) Application number: **87309557.4**

(22) Date of filing: **29.10.87**

(54) **Monoclonal antibody, process for preparation thereof and diagnostic drug containing the same.**

(30) Priority: **31.08.87 JP 218149/87**

(43) Date of publication of application:
**08.03.89 Bulletin  89/10**

(45) Publication of the grant of the patent:
**03.03.93 Bulletin  93/09**

(84) Designated Contracting States:
**DE FR GB**

(56) References cited:

CHEMICAL ABSTRACTS, vol. 95, no. 7, 17th
August 1981, page 485, no. 59448s, Colum-
bus, Ohio, US; S. VORA et al.: "Assignment
of the human gene for liver-type
6-phosphofructokinase isozyme (PFKL) to
chromosome 21 by using somatic cell hy-
brids and monoclonal anti-L antibody", &
PROC. NATL. ACAD. SCI. U.S.A. 1981, 78(6),
3738-42

(73) Proprietor: **Shin-Etsu Chemical Co., Ltd.**
**6-1, Otemachi 2-chome**
**Chiyoda-ku Tokyo 100(JP)**

(72) Inventor: **Onodera, Kazukiyo**
**62-1-809, Nakajuku Itabashi-ku**
**Tokyo(JP)**
Inventor: **Obata, Ranko**
**1-599, Kosugijinya-cho**
**Nakahara-ku Kawasaki-shi**
**Kanagawa-ken(JP)**
Inventor: **Hongu, Tatsuhiko**
**3-23-3, Shimoda-cho Kohoku-ku**
**Yokohama-shi Kanagawa-ken(JP)**
Inventor: **Guterres, Gilberto M.A.**
**9-40-13-101, Takashimadaira Itabashi-ku**
**Tokyo(JP)**

(74) Representative: **Votier, Sidney David et al**
**CARPMAELS & RANSFORD 43, Bloomsbury**
**Square**
**London WC1A 2RA (GB)**

## Description

The present invention relates to a monoclonal antibody which specifically binds to a cell membrane component comprised of at least one protein encoded by human chromosome No. 21, a process for preparation thereof, and a diagnostic drug for Down's syndrome containing the antibody.

Recently, hereditary and nonhereditary congential chromosomal abberation diseases have been increased, and this tendency has become a worldwide phenomenon. Medical approaches against these abberation diseases mainly depend on analysis of exanthrope up to recently, and analysis and test method thereof on hereditary factors, particularly chromosomal aberration have been extremely limited. This is because since chromosomes cannot be observed in ordinal cells and can only be observed in cells in the mitotic period, special techniques are required for fixing method thereof. Therefore, early diagnosis, particularly prenatal diagnosis of chromosomal aberrations is a social demand, and establishment of easy and highly confidential diagnostic methods therefor has been desired.

Though many of fertilized eggs having any chromosomal aberration are naturally selected during early phase of pregnancy, about one percent of born children are born in a state having the aberration, and thus many of them have malformations and/or intensive disturbances in anima development. Kind of chromosomal aberration is classified into aberration in number and aberration in structure. Triploid formed by fertilizing one egg with two sperms does not grow even if it is born, whereas since a patient of trisomy wherein the same chromosome excessively exists in the nucleus grows accompanying mental and physical aberrations, wrestling therewith from whole society has been desired. 21-Trisomy having the chromosome No. 21 in an excess number of one causes Down's syndrome (hereinafter simply referred to as "Down's disease"). Natality of children of Down's disease tends to increase in accordance with recent increase of marriage in high age, and thus methods for early diagnosis prior to childbirth have been desired.

One object of the present invention lies in providing novel monoclonal antibodies useful for diagnosing, in simple and high confidence, Down's disease caused by chromosome No. 21 having aberration in number, a process for preparation thereof, and a diagnostic drug containing said monoclonal antibody.

The present inventors have succeeded in obtaining monoclonal antibodies capable of recognizing in a high sensitivity the aberration of human chromosome No. 21 in number by applying known cell fusion techniques. Furthermore, they have developed a diagnostic drug useful for diagnosis of Down's disease by utilisation of the monoclonal antibodies.

It has been discovered that at least one membrane protein, among the many such proteins encoded by genes located on human chromosome No. 21, is produced by normal (diploid) cells, such as fibroblasts, in substantially lesser amounts than by cells containing an aberrant number of chromosome No. 21, as is the case for Down's disease patients. It has also been found that, in accordance with the present invention, monoclonal antibodies can be reproducibly obtained that recognise chromosome 21-polysomic cells by virtue of binding to a membrane component that is present, at detectable levels, in such polysomic cells but not in normal cells.

Monoclonal antibodies of the present invention are therefore useful in the diagnosis of Down's disease caused by trisomy-21, i.e., they specifically bind to a protein-containing membrane component present in detectable amounts only when human chromosome No. 21 is polysomic. These monoclonal antibodies are therefore able to distinguish between normal cells and cells that are, for example, trisomic for chromosome No. 21.

A monoclonal antibody of the present invention can be obtained, for example, by a process comprising steps of immunising an animal with a Chinese hamster ovary cell into which human chromosome No. 21 has been introduced and which has a membrane protein encoded by the human chromosome No. 21; fusing antibody-producing cells taken out from said animal with a myeloma cell to form hybridomas; subjecting the hybridomas to selection, screening and cloning to obtain a hybridoma capable of producing a monoclonal antibody which specifically recognises a membrane component of cells where human chromosome No. 21 is present in an aberrant number; and culturing the hybridoma.

The Chinese hamster ovary cell (CHO cell) into which human chromosome No. 21 has been introduced and which has the membrane protein encoded by human chromosome No. 21 used in the above process may be obtained by known cell fusion techniques. An example of such CHO cell includes so-called 2Fu$^r$ - [see D. Patterson et al, Som. Cell Genet., 1, 91-110, (1975)].

Examples of the animals to be immunised as used in the above process include mouse, rat and the like, and mouse is usually used.

Route and schedule of administration of the antigen into the animal may variously be varied. For example, according to a preferred administration method, the antigen is first treated with mitomycin and intraperitoneally administered by several portions to the animal. Antibodies are produced in the spleen of the animal by administration of the antigen. As antibody-producing cells, spleen cells are usually used.

As the myeloma cell to be used with the thus obtained antibody-producing cells, a cell induced from BALB/C mouse MOPC 21 myeloma (as referred to as NS-1) is, for example, used. This NS-1 has resistance to 8-azaguanine, and dies in a medium containing hypoxanthine-aminopterin-thymidine (HAT medium) since it lacks the enzyme hypoxanthine guanine phosphoribosyltransferase. Therefore, use of NS-1 is convenient for selective culture of hybridomas to be obtained. As a fusing agent for cell fusion, polyethylene glycol is usually used. The above-mentioned cell fusion techniques, myeloma cells to be used, and the like are known, per se. [myeloma cells; Kohler et al, Fur. J. Immunol., 6, 292-295, (1976), and fusing method; Kohler et al, bid ., 6, 511-516, (1976)].

The resulting hybridomas are screened to select those which can produce antibodies capable of binding to the CHO cell used for the immunisation as stated above and to a cell of a human being but incapable of binding to a normal CHO cell. This screening can be conducted in a conventional manner. The kind of the cell of human being used for the screening is not limited. The hybridomas thus screened are then cloned, whereby progenies of respective hybridomas from respective phyletic lines are obtained. Monoclonal antibodies that can recognise a membrane protein characteristic of cells where chromosome No. 21 is present in aberrant number can successively be synthesised and secreted by respectively infinitely growing these cell lines, i.e. clones in a cell culture medium (in vitro culture) or in vivo. Each of the monoclonal antibodies thus produced can be recovered according to a method known in the art.

The monoclonal antibody thus obtained specifically binds, when a fibroblast cell is targeted, for example , to a protein component of the cell membrane produced in detectable amounts when chromosome No. 21 is trisomic rather than disomic.

The present invention also provides a diagnostic drug for Down's disease owing to 21-trisomy containing the above-mentioned monoclonal antibody. Aberration of chromosome No. 21 in number can readily be determined by applying this diagnostic drug, for example, to human fibroblasts or the like. Therefore, diagnosis of Down's disease can readily be carried out by use of this diagnostic drug. The diagnostic drug of the present invention can practically be used through any immunological test methods, for example, immunofluorescence, an immunoadhesionhemocyte-agglutinating reaction method, radioimmunoassay or the like. For example, in utilisation of immunofluorescence, human cells are treated with the monoclonal antibody of the present invention, then reacted with an anti-mouse IgG labelled with a fluorescent dye such as FITC, and set in a fluorescence microscope, and the state of luminescence is checked, whereby presence or absence of chromosomal aberration can readily be diagnosed.

Monoclonal antibodies and diagnostic drug of the present invention are further explained below according to non-restricting examples.

## EXAMPLES

Example 1

(1) Production of antibodies

CHO cells ($2Fu^r$) into which human chromosome No. 21 had been introduced and which had the membrane protein encoded by the chromosome No. 21 was treated with mitomycin C, and washed with PBS (phosphate buffer). A $2Fu^r$ suspension of $5 \times 10^6$ cells/0.5 cc in PBS was intraperitoneally injected into a female BALB/C mouse (immunisation). Two weeks thereafter, a $2Fu^r$ suspension of $1 \times 10^7$ cells/0.5 cc in PBS and further two weeks thereafter, a $2Fu^r$ suspension of $1 \times 10^7$ cells/0.5 cc in PBS were injected in the same manner as above. Three days thereafter, the spleen of the mouse was taken out, finely pulverised in RPMI-1640 medium, subjected three times to centrifugation (1500 rpm) for washing, and resuspended in the medium.

Sample of cultures of $2Fu^r$ used above can be obtained upon written request to Shin-Etsu Chemical Co. Ltd., 6-1, Ohtemachi 2-chome, Chiyoda-ku, Tokyo, Japan.

(2) Cell fusion

$1.1 \times 10^8$ cells of the spleen cell obtained in the above procedure and $1.0 \times 10^7$ cells of BALB/C mouse myeloma cell (NS-1) once washed in advance with RPMI-1640 medium were mixed, and centrifuged at 1500 rpm for 5 minutes to prepare a pellet. Then, 1 ml of a solution obtained by dissolving polyethylene glycol (1540) in RPMI-1640 medium to 50 wt% was added to the pellet with stirring, RPMI-1640 medium was added thereto to the total volume of 10 ml, and the mixture was stirred for 6 minutes. The mixture was subjected to centrifugation at 1000 rpm for 5 minutes to remove polyethylene glycol, and the solid matters were resuspended in 40 ml of RPMI-1640 medium containing 20 vol% of fetal calf serum (FCS).

(3) Selection, screening and cloning

0.2 ml (5 × 10⁶ cells) of the above suspension was placed in each of 192 wells of a 96 wells-plate for tissue culture, and half amount of the culture supernatant was replaced every 3 to 4 days with HAT medium (136.1 mg/ml hypoxanthine, 1.76 mg/ml aminopterine and 38.75 mg/ml thymidine). The culturing condition was a temperature of 37°C, a humidity of 100% and $CO_2$ gas concentration of 7%. 10 days after the start of the culturing, hybridomas were observed in 119 wells (emergence rate: 62%). Culture supernatants in the 119 wells, which revealed positive results, were subjected to screening by checking according to enzyme-labelled antibody techniques (ELISA) using 2Fuʳ whether or not the supernatants contain antibodies, and the results revealed 20 wells to be positive (10.6% of the total wells).

Cells in each of the 20 wells were subjected to limiting dilution using HAT medium with supplementation of BALB/C mouse thymocyte of $1.0 \times 10^7$ cells/ml, 0.2 ml portions of the resulting cell suspensions were placed in each well of a 96 wells-plate for tissue culture, and culturing was carried out. In this connection, hybridoma number per well and well number were 48 wells of 1 cell/well, 45 wells of 5 cells/well and 3 cells of 20 cells/well. Culturing conditions were the same as above. The culture supernatants were respectively subjected to the same ELISA as above-mentioned to screen those which are negative for the CHO cell and positive for human leukemia cell (TALL), and as the result desired clones were obtained from three wells. The cells in the three wells were respectively recloned two times according to the limiting dilution method, and the resulting hybridomas were respectively named OK-1, OK-2 and OK-3.

It has been found by an enzyme-labelled antibody technique using an antimouse antibody that monoclonal antibodies produced from hybridomas OK-1, OK-2 and OK-3 belong to the classes IgM, IgG and IgM, respectively.

Furthermore, protein to which the monoclonal antibodies produced by OK-1, OK-2 and OK-3 respectively recognise and bind are as follows.

- Monoclonal antibody produced by OK-1

This antibody recognises four bands in Western blotting of human leukemia cell (TALL) protein.

- Monoclonal antibody produced by OK-2

This antibody recognises bands of 40 and 90 kilodaltons in Western blotting of TALL protein.

- Monoclonal antibody produced by OK-3

This antibody recognises four bands of 86.4, 74.2, 61.4 and 36.4 kilodaltons in Western blotting of TALL protein, and recognises three bands of 89, 82 and 45 kilodaltons in Western blotting of 2Fuʳ protein.

Example 2

Diagnosis example by detecting the protein according to immunofluorescence

Fibroblasts of a normal human being and a patient of Downs' disease were respectively placed together with a cover glass (12 × 12 mm) for a microscope in a Petri dish having a diameter of 35 mm, and cultured. As the medium was used a mixture wherein 10% nonessential amino acid (NEAA), 10 mM sodium pyruvate, 0.1% lactoalbumin hydrolyzate (LAH) and 10% FCS were contained in MEM. The cells were respectively cultured at room temperature for 5 days, fixed in a 3.7% formaldehyde solution in PBS immediately after washing the cover glass 2 to 3 times with PBS, washed with distilled water, and further fixed in acetone at -20°C.

15 μl of the monoclonal antibody obtained in Example 1 (antibody derived from hybridoma OK-1, OK-2 or OK-3) was placed on the fixed cells, and the mixture was allowed to react at room temperature for one hour. After washing the mixture three times with PBS, 15μl of antimouse IgG antibody or an antimouse IgM antibody, as a second antibody, respectively labelled with FITC was placed thereon, the mixture was allowed to react at room temperature for one hour. The mixture was washed three times with PBS, once washed with distilled water in order to remove salts, placed on a slide glass at a state of the surface fixing the cells down, and sealed with Gelvatol to obtain a test sample piece.

The test sample was tested about presence of luminescence on the surface layer using a fluorescence microscope. The resulting results are shown in Table 1. Fibroblasts of the patient of Down's disease emitted fluorescence, whereas those of the normal human being did not emit. Thus, it will be appreciated that presence of chromosomal aberration can readily be diagnosed in a high sensitivity without using a special technique according to the present invention.

In order to confirm the existence of the protein on the membrane , detection by membrane immunofluorescence wherein the cells are not fixed and reacted with a fluorescent dye was also carried out as paralleled to the above method, and the resulting results were the same as those obtained above.

Table 1   Presence or absence of luminescence by
          immunofluorescence

| Monoclonal antibody | Fibroblast | |
|---|---|---|
| | Normal human being | Patient of Down's disease |
| Produced by OK-1 | – | + |
| Produced by OK-2 | – | + |
| Produced by OK-3 | – | + |

Note +: Luminescence was observed
     -: Luminescence was not observed

The hybridomas OK-1, OK-2 and OK-3 were deposited with the Fermentation Research Institute, Ibaraki-ken, Japan on October 20th, 1987.

**Claims**

1. A monoclonal antibody which specifically binds to a membrane component comprising a protein encoded by human chromosome No. 21 where human chromosome No. 21 is present in an aberrant number produced by hybridoma cell line FERM BP-1802 or FERM BP-1803, or by the progeny or a mutant thereof.

2. A monoclonal antibody according to Claim 1, wherein said monoclonal antibody distinguishes normal human cells from polysomic human cells.

3. A monoclonal antibody according to Claim 2, wherein said monoclonal antibody distinguishes normal human fibroblasts from fibroblasts that are trisomic for human chromosome No. 21.

4. A process for preparing the monoclonal antibody according to Claim 1, which comprises the steps of immunising an animal with a Chinese hamster ovary cell into which human chromosome No. 21 has been introduced and which expresses at least one membrane protein encoded by the human chromosome No. 21; fusing antibody-producing cells taken out from the animal with a myeloma cell to form hybridomas; subjecting the hybridomas to selection, screening and cloning to obtain a hybridoma capable of producing a monoclonal antibody which specifically binds to a membrane component comprising a protein encoded by human chromosome No. 21; and culturing the hybridoma.

**5.** A diagnostic reagent for Down's disease, comprising a monoclonal antibody according to claim 1.

**6.** A diagnostic reagent as claimed in claim 5, wherein the said monoclonal antibody distinguishes normal fibroblasts fom fibroblasts that are trisomic for human chromosome No. 21.

**7.** Hybridoma cell line FERM BP-1802 or FERM BP-1803, or the progeny or a mutant thereof which expresses an antibody having the specificity defined in claim 1.

**Patentansprüche**

**1.** Monoklonaler Antikörper mit spezifischer Bindung an eine ein Protein umfassende Membrankomponente, wobei das Protein durch humanes, in abweichender Zahl vorliegendes Chromosom Nr. 21 codiert ist, produziert durch die Hybridom-Zellinie FERM BP-1802 oder FERM BP-1803 oder durch einen Nachkommen oder eine Mutante davon.

**2.** Monoklonaler Antikörper nach Anspruch 1, worin der monoklonale Antikörper zwischen normalen, humanen Zellen und polysomalen, humanen Zellen unterscheidet.

**3.** Monoklonaler Antikörper nach Anspruch 2, worin der monoklonale Antikörper zwischen normalen, humanen Fibroblasten und Fibroblasten unterscheidet, die für humanes Chromosom Nr. 21 trisomal sind.

**4.** Verfahren zur Herstellung des monoklonalen Antikörpers nach Anspruch 1, umfassend die folgenden Schritte:
- Immunisierung eines Tieres mit einer Ovariumzelle eines Chinesischen Hamsters, in die humanes Chromosom Nr. 21 eingeführt worden ist und die mindestens ein durch das humane Chromosom Nr. 21 codierte Membranprotein exprimiert,
- Fusion von aus dem Tier entnommenen, Antikörperproduzierenden Zellen mit einer Myelomzelle zur Ausbildung von Hybridomen,
- Selektionierung, Screening und Klonierung der Hybridomen zum Erhalt eines Hybridom, das zur Produktion eines monoklonalen Antikörpers fähig ist, der spezifisch an eine Membrankomponente bindet, die ein durch humanes Chromosom Nr. 21 codiertes Protein umfaßt, und
- Kultivierung des Hybridoms.

**5.** Diagnostisches Reagens für das Down-Syndrom, umfassend einen monoklonalen Antikörper nach Anspruch 1.

**6.** Diagnostisches Reagens nach Anspruch 5, worin der monoklonale Antikörper zwischen normalen Fibroblasten und Fibroblasten unterscheidet, die für humanes Chromosom Nr. 21 trisomal sind.

**7.** Hybridom-Zellinie FERM BP-1802 oder FERM BP-1803 oder ein Nachkomme oder eine Mutante davon, die einen Antikörper mit der in Anspruch 1 angegebenen Spezifität exprimieren.

**Revendications**

**1.** Anticorps monoclonal qui se fixe spécifiquement sur un constituant de membrane comprenant une protéine encodée par le chromosome humain n° 21, lorsque le chromosome humain n° 21 est présent en un nombre aberrant anticorps produit par une lignée de cellules d'hybridome FERM BP-1802 ou FERM BP-1803,ou par une descendance ou un mutant de cette lignée.

**2.** Anticorps monoclonal selon la revendication 1 ,dans lequel ledit anticorps monoclonal distingue des cellules humaines normales de cellules humaines polysomiques .

**3.** Anticorps monoclonal selon la revendication 2, ledit anticorps monoclonal distinguant des fibroplastes humains normaux de fibroplastes qui sont trisomiques pour le chromosome humain n° 21 .

**4.** Procédé pour préparer l'anticorps monoclonal selon la revendication 1 , qui comprend les étapes consistant à immuniser un animal avec une cellule d'ovaire de hamster de Chine, dans laquelle du

chromosome humain n° 21 a été introduit,et qui exprime au moins une protéine de membrane encodée par le chromosome humain n° 21 ; à provoquer une fusion de cellules productrices d'anticorps,prélevées sur l'animal, avec une cellule de myélome pour former des hybridomes ;à soumettre les hybridomes à sélection, criblage et clonage pour obtenir un hybridome capable de produire un anticorps monoclonal qui se fixe spécifiquement sur un constituant de membrane comprenant une protéine encodée par le chromosome humain N°21 ; et à cultiver l'hybridome.

5. Réactif diagnostique pour déceler la maladie de Down ,comprenant un anticorps monoclonal selon la revendication 1 .

6. Réactif diagnostique tel que revendiqué à la revendication 5 ,ledit anticorps monoclonal distinguant des fibroblastes normaux de fibroblastes qui sont trisomiques pour le chromosome humain N°21.

7. Lignée de cellules d'hybridome FERM BP-1802 ou FERM BP-1803,ou la descendance ou un mutant de cette lignée,qui exprime un anticorps ayant la spécificité définie à la revendication 1 .